# EUROPEAN PATENT APPLICATION

(11) **EP 1 978 083 A2**
(43) Date of publication of application: **08.10.2008**
(21) Application number: 08425088.5
(22) Date of filing: 13.02.2008
(51) Int. Cl.: C12G 1/02, C12G 1/06, C12N 1/18, C12R 1/865, C12R 1/85

(54) **Yeasts for champagnization**

(30) Priority: 16.02.2007 IT RM20070080
(71) Applicant: Parco Scientifico e tecnologico della sicilia, Z.I. Blocco Palma I Stradale Agnelli Ang. Via Lancia 95030 Catania (IT)
(72) Inventor: Lombardo, Alessandro, 95030 Catania (IT); Guarrera, Nicola, 95030 Catania (IT); Paparone, Nicoletta, 95030 Catania (IT)
(74) Representative: Gitto, Serena

(57) **Abstract**

The present invention concerns new yeast strains belonging to *Saccharomyces bayanus* and *Debaryomyces hansenii* species and their use, separately or in combination, in fermentation processes, i.e. champagnisation.

## Description

The present invention concerns two new yeast strains belonging to *Saccharomyces bayanus* and *Debaryomyces hansenii* species and their use, separately or in combination, in a fermentation or re-fermentation process, to obtain fermented or re-fermented drinks. Particularly, the present invention refers to a champagnisation process to obtain spumante..

Spumantes are special wines which at the end of the normal white or rosé vinification are subjected to another fermentation resulting in peculiarities of spumantes: the high pressure within the bottle (minimal 3,5 atmospheres) provokes the froth when the stopper is blown and the carbon dioxide originates very small characteristic bubbles in the glass, i.e. "perlage".

Spumantes are very valuable wines necessitating a fine and laborious working. The choice of the grapes and preparation of the base wine must be most accurate in order to exalt the freshness and bouquet.

The grape harvest time is chosen based on the optimal acidity grade of the grape and therefore it is often anticipated in comparison to "the smooth" whites. The vinification, as first, and champagnisation as second, processes must be carried out at not high temperatures. The spumantes can be produced according to the classic Champenois or Charmat methods.

The classic method essentially consists of favouring a second wine fermentation occurring within a bottle, a process that is named bottle re-fermentation. A particular aspect of this technique is just characterised by the bottle that is associated to the wine from re-fermentation to the consumption time. In other words the bottle containing a classic method spumante is exactly the same as for the production thereof.

The transformation of the wine in classic method spumante begins with the addition to the *cuvée* of a mixture, named liqueur de tirage, consisting of base wine, cane sugar, selected yeasts and other substances useful to facilitate the rémuage process. In most cases the addition of 24 g/l of sugar are sufficient to result in a 6 atmospheres pressure (4 g of sugar develop, during the fermentation, approximately 1 atmosphere) inside the bottle and for this reason the thickness of spumante bottles is greater than others so as to prevent outbreak thereof.

Following bottling and capping the bottles are stored in horizontal position in wine cellar at a constant temperature of approximately 10°C and during this period inside the bottle begins the fermentation by the yeasts. Thus begins the prise de mousse, i.e. the true wine re-fermentation. In this period the carbon dioxide, not being possible to exit from the bottle, remains "trapped" inside thereof thus generating fizzing properties. The re-fermentation has a duration of approximately one or two months and at the end thereof the wine alcoholic degree will be increased by approximately 1.2-1.3°. During the re-fermentation the bottles are periodically rotated so as to avoid the incrustation of yeasts on the wall of the bottle. When the re-fermentation is finished, that is to say when all the sugar has been transformed by yeasts into alcohol and carbon dioxide, the bottles come left to mature in wine cellar and it is in this slow step that the wine will increase its organoleptic complexity. At this point yeasts begin to release aromatic substances into the wine, amino acids, proteins and volatile compounds, which will enrich the wine qualities. This process is named yeasts autolysis and generally it occurs 8-10 months after the end of the re-fermentation.

The quality of spumante and organoleptic properties thereof are strongly affected by selected yeast strain driving the prise de mousse within the bottle. Various yeast strains have specific different characteristics through a peculiar individual metabolism, affecting not only the quality, but also the typicality intensity of obtained spumante. In fact when the must is subjected to the fermentation and subsequently to re-fermentation, the yeast acts on it by degrading and transforming most of the organic substances characterising the composition thereof.

The result of the re-fermentation will depend therefore not only on the characteristics of the base wine but also on the yeast: strain; in other words, starting from base wines with same compositions, and carrying out the re-fermentation using different yeast strains, differently tasting spumantes are obtained; similarly a unique yeast strain, used for the re-fermentation of two different base wines will result in two different spumantes.

During the prise de mousse the yeast, if contains necessary enzymes, can act on the base wine by transforming the already present and typical aromatic substances of the specific grapevine, releasing sugar bound, and therefore not active, substances, and producing new aromatic substances by metabolising sugars and nitrogenous substances.

In the selection of the yeast more suitable to enhance the quality and typicality of a spumante, not only the yeast genetic characteristics and therefore its ability to modify the composition of the base wine, but also the ability to carry out complete re-fermentations at low temperatures must be taken into account.

Other yeast characteristics, important in that they affect the final quality of the spumante, are: produced amounts of acetic acid and hydrogen sulphide, the beta-glucosidase presence which can release glycosylated aromatic substances.

In the light of above reported, it is apparent the interest to select new yeast strains which can be used advantageously in the re-fermentation processes, i.e champagnisation, in order to obtain a fermented drink with high quality from the point of view of the aromatic content and contribute to better define the typicality of the product, that is the identity of a sparkling spumante wine deriving from a particular geographic area.

The authors of the present invention now provided a re-fermentation and/or autolysis improved process by means of the use, alone or in combination, of two new yeast strains having metabolic characteristics suitable to obtain spumantes or other fermented drinks with excellent quality and aroma intensity. The quality of a food product, considered not only as alimentary safety but also as attainment of the total consent by the consumer is a factor increasingly associated to the concept of "flavour", that is, indissoluble interaction among tactile impression, taste and aroma (Steinhart et al. 2000).

In order to estimate the olfactively active fraction, i.e. the part of the spumante aromatic component smelled by human nose, the authors used the gas-chromatography/olfactometry technique (GC/O) (Dravnieks and O'Donnell, 1971; Gain *et al.,* 1996). This particular technique, by splitting up the exit flow from a gas-chromatographic column to a standard detector (FID) and opportunely trained operator (sniff-detector), allows to distinguish eluted active molecules as olfactively odorous and not

The fundamental points in the olfactometric analysis are based on the choice of the method for the isolation of the volatile fraction from the examined alimentary matrix and adequate training and composition of the olfactometric panel. In order to obtain an exhaustive representation of the aromatic-olfactive profile of the food, olfactometric obtained results have been supported by a quantitative determination of the entire aromatic fraction and crossed with the data obtained from a sensory panel (dilution, intensity and frequency methods)

It is therefore an object of the present invention *Saccharomyces bayanus* yeast strain, DSM deposit No. 18477 (deposited at DSMZ on 20 July 2006).

It is a further object of the present invention *Debaryomyces hansenii* yeast strain DSM deposit No. 18478 (deposited at DSMZ on 20 July 2006).

In addition the present invention refers to association of above said two yeast strains, *Saccharomyces bayanus* DSM18477 and *Debaryomyces hansenii* DSM18478.

Alternatively, the present invention contemplates their combination with a commercially available yeast strain. In a preferred embodiment of the invention said strain is *Saccharomyces bayanus* when in association with *Debaryomyces hansenii* DSM18478 and viceversa *Debaryomyces hansenii* when in association with *Saccharomyces bayanus* DSM18477 (i.e. *Saccharomyces bayanus* EC1118 + *Debaryomyces hansenii* DSM18478).

Further the invention concerns the use of at least one of the two yeast strains *Saccharomyces bayanus* DSM18477 and *Debaryomyces hansenii* DSM18478 or combination thereof or another commercially available yeast strain (i.e. *Saccharomyces bayanus* EC1118) in an autolysis and/or re-fermentation process to obtain re-fermented drink. Preferably, said process is champagnisation and said obtained fermented drink is spumante.

According to an alternative embodiment the invention refers to the use of at least a *Saccharomyces bayanus* DSM18477 yeast strain or a yeast combination as above defined, in a fermentation process to obtain a fermented drink selected from the group consisting of cider, fruit juice, beer, wine, raisin wine and sake.

The invention further concerns a mixture (liqueur de tirage) comprising the following components:
- at least one of the two *Saccharomyces bayanus* DSM18477 and *Debaryomyces hansenii* DSM18478 yeast strains or their combination or with another commercially available yeast (i.e. *Saccharomyces bayanus* EC1118);
- sugar, preferably ; sugar cane
- base wine.

Preferably, said sugar is at a concentration of 20-25 g/l, preferably 24 g/l. The mixture can further comprise other substances useful to favour the rémuage process.

The present invention concerns a fermentation process to obtain a re-fermented drink, more preferably a champagnisation process to obtain spumante, comprising the steps of bottle re-fermentation and autolysis by using a mixture comprising at least a yeast strain, base wine and sugar (preferably cane sugar) at a temperature from 8°C to 16°C, preferably 10°C, for at least 8-10 months; wherein said mixture (i.e liqueur de tirage) as above defined comprises:
- at least one of the two *Saccharomyces bayanus* DSM18477 and *Debaryomyces hansenii* DSM18478 yeast strains or their combination or with another commercially available yeast strain (i.e. *Saccharomyces bayanus* EC1118);
- sugar, preferably sugar cane;
- base wine.

Finally, the invention refers to a re-fermented drink, preferably a spumante, obtainable using the fermentation process as above defined, which, in the preferred embodiment of the present invention, is a champagnisation process.

According to a further aspect the present invention refers to a fermentation process to obtain a fermented drink, characterised in that it comprises a fermentation step of a fermentable base using at least a *Saccharomyces bayanus* DSM18477 yeast strain or yeast combination as above defined, to obtain a fermented drink, selected from the group consisting of cider, fruit juice, beer, wine, raisin wine and sake. Preferably, said fermentable base is selected from the group consisting of malt (from cereals, wheat, barley), must (from grape, strawberry grape), oat, fruit juice (i.e prune), rice.

The invention further refers to a fermented drink obtainable through the fermentation process which employs yeasts alone or in association according to the present invention.

The present invention now will be described by illustrative, but not limitative way, according to preferred embodiments thereof, with particular reference to enclosed drawings, wherein:
the figure 1 shows the analysis of the aromatic content (total area) of 4 analysed samples witness T1, T2, T3 (Witness: EC1118, commercially available yeast *S. bayanus,* Lallemand, Canada; T1: DSM18477; T2: DSM18477+DSM18478; T3: EC1118+DSM18478); in addition table illustrating the percentage distribution of the aromatic components detected in said four samples is reported;
figure 2 shows fAFLP profiles identifying yeast strains according to the invention, i.e. *Saccharomyces bayanus* DSM18477 (deposited at DSMZ on 20 July 2006) and *Debaryomyces hansenii* DSM18478 (deposited at DSMZ on 20 July 2006);
figure 3 shows two tables indicating respectively concentrations (ppm) of the components quantifiable using external standards (Table 4A) and percentage distribution of not quantifiable components using external standards (Table 4b) in said 4 samples, witness, T1, T2, T3;
figure 4 shows table 5 indicating detected odorous components, associated descriptor, and persistence and intensity values in said 4 samples, witness, T1, T2, T3;
figure 5 shows table 6 indicating AUC values of olfactively active compounds in said 4 samples, witness, T1, T2, T3;
figure 6 shows table 7 indicating the effect of samples (4), panel components (10), replicates (3) and relevant interactions on 17 descriptors;
figure 7 shows table 8 indicating multiple range test on the average values of the 17 sensory descriptors;

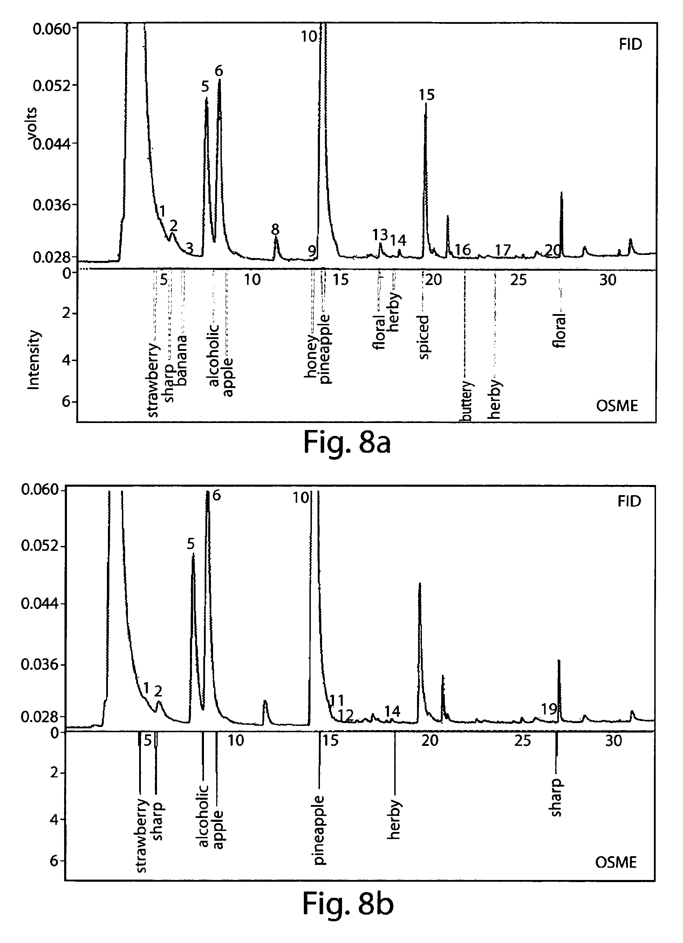

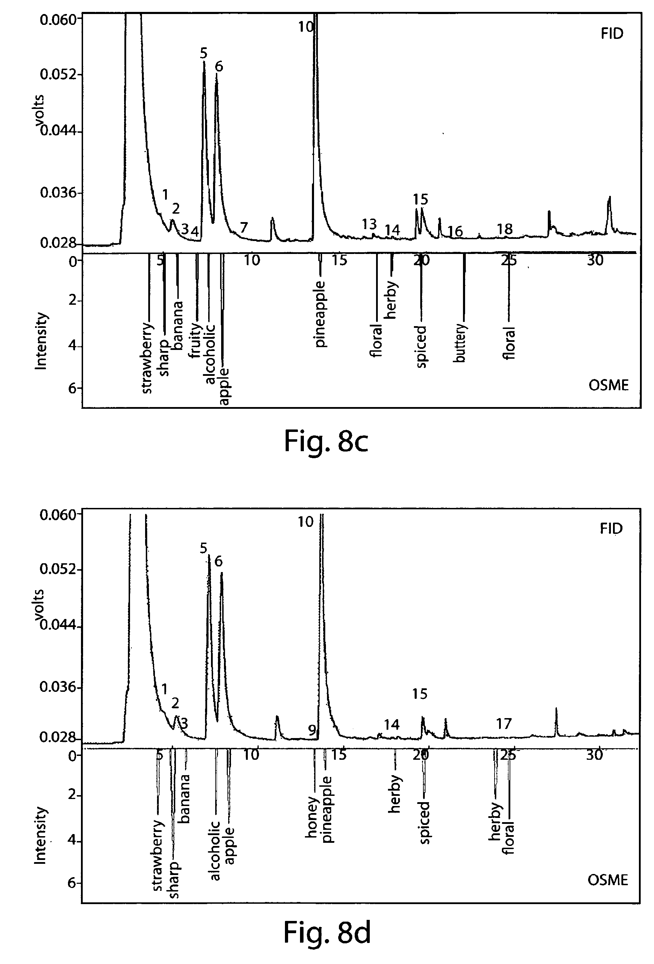

figure 8 shows osmograms of said 4 samples: Witness (panel a); T1 (panel b); T2 (panel c); T3 (panel d).

### EXAMPLE 1: Isolation, selection and characterisation of said two yeast strains for the improvement of spumante produced according to the classic method

### MATERIALS AND METHODS

### Isolation of strains from fermenting grapes

Used yeast strains have been isolated from Lipari fermenting malvasia grapes. Particularly, *Debaryomyces hansenii* DSM18478 (deposited at DSMZ on 20 July 2006) has been isolated during the first period of the fermentation, while *Saccharomyces bayanus* DSM18477 (deposited at DSMZ on 20 July 2006) has been isolated at the end of the spontaneous alcoholic fermentation. Fermenting must aliquots, opportunely diluted by decimal series, have been seeded on YPD complete medium (Roses et al., 1990). After 2 days of incubation at 26°C, representative colonies have been withdrawn, spread on the same medium and incubated for two days. Pure cultures have been stored at 4°C and renewed periodically.

### Biochemist-physiological characterisation of strains

Isolated yeast strains have been analysed with respect to following characteristics: capacity of spore generation on sporulation medium (Roses et al. 1990); use of lysine as unique nitrogen source in lysine medium (OXOID, Basingstoke, UK); fermentation of sugars (galactose, saccharose, maltose, raffinose and melibiose) (Mortimer et al., 1994); resistance to cyclohexylimide (3 and 800 mg/l) on medium containing 0.67 % YNB (Sigma, St. Louis, USA) and 1% glucose; tolerance to 1% acetic acid as reported by Van der Walt and Yarrow (1984); production of H₂S in Biggy agar medium (OXOID, Basingstoke, UK); production of β-glucosidase on agar treated medium containing arbutine (5 g/l), peptone (10 g/l), yeast extract (3 g/l), glucose (1 g/l), ferric citrate (0,5 g/l), minimal inhibiting sodium metabisulfite concentration on agar must medium (pH 3.2).

### Microvinification tests

Microvinification tests have been carried out in 100 ml flasks containing 80 ml of sterilised must from nerello mascalese sterilised for 30 min with flowing vapour, with approximately 23% of sugars.

### Molecular characterisation of yeast strains named DSM18478 and DSM18477 by means of fluorescence AFLP and ribotyping

DNA ribotyping has been carried out as reported by McCullough et al. (1998).

Fluorescence AFLP technique is based on a selective amplification of restriction fragments from a complete digestion of genomic DNA and detection of amplified products by means of capillary electrophoresis carried out using a completely automated analytical system. The technique consists of three steps: genomic DNA restriction and ligation to oligonucleotide adaptors; selective amplifications of restriction fragment sub-set using marked primers in fluorescence conditions; capillary electrophoresis of amplified fragments. Amplified restriction fragments are generate from genoma unique loci and originate DNA polymorphism. Fragments length differences can be determined from the changes of nucleotide bases in the restriction or extension site of the primer or by insertion or deletion in the body of DNA fragment. The peak number detectable by an AFLP reaction is very high and it is generally necessary to adapt selective PCR in order to select a such fragment number that analysis by the genetic analyser is possible. The fluorescence AFLP is suitable to generate fingerprinting characteristic of the species under study and it is highly reliable and reproducible, moreover different primer combinations used in order to amplify different restriction fragment sub-set produce different fingerprinting. Fluorescence capillary electrophoresis has high analytical capacity and allows great sample number to be analysed at the same time. Therefore it is possible highly resolved fingerprinting to be obtained using a procedure more reliable and faster than conventional one carried out using radioactive polyacrylamide gel (Dresler-Nurmi et al., 2000). fAFLP technology is enough sensitive to result in low levels of genetic variability allowing highly correlated species to be discriminated.

Yeast fingerprinting obtained by means of conventional AFLP and in fluorescence conditions have been obtained in order to study the genetic variability of clinical and commercially available isolates (Barros Lopes *et al.,* 1999; Borst *et al.,* 2004). The determination of genomic similarity among yeast strains implies the possibility isolates of agro-industrial interest certainly and shortly to be discriminated and identified.

### DNA extraction

A *Debaryomyces hansenii* (DSM 18478) and a *Saccharomyces bayanus* (DSM18477) strains have been inoculated in 3 ml YPD broth and incubated o/n at 28°C under agitation. DNA has been subsequently extracted according to the protocol proposed by Guthrie and Fink (1991). Then DNA presence has been verified on 1 % agarose gel in TBE 0,5 x stained with 10 mg/ml Ethidium Bromide. The quantification has been then carried out by spectrophotometry readings at 260 nm (Biophotometer, Eppendorf).

### Restriction and ligation

DNA isolated from strains under study has been digested and ligated in a single reaction; 500 ng of purified genomic DNA have been added to a mixture containing: 1x buffer T4 DNA ligase, NaCl 0.05 M, BSA 0.045 mg/ml, *EcoRl* adaptors 1 µM and *Msel* adaptors 5 µM, *EcoRl* 5U, *Msel* 5U, T4 DNA ligase (Invitrogen), to 30 µL final volume. The adaptors have been heated for 5 min at 95°C and cooled at room temperature for at least 10 min before the use. The whole has been incubated o/n at 37°C in thermostatic bath. The reaction product has been stained on 1.2% agarose gel (Agarose D-1 low EEO, Pronadisa CONDA) containing Ethidium Bromide. The reaction product has been diluted x 10 with TE 10:1 buffer.

### Pre-selective amplification

A mixture containing 1 x PCR buffer, 0.2 mM dNTP, 2.0 milimeter MgCl₂, 0.3 µM *EcoRI* primer (+1) and 0.3 µM *Msel* primer (+1) and 0.5 U of Taq recombinant polymerase (Invitrogen), 3 µl of diluted restricted/ligated sample to 13 µl final volume. PCR schedule was as below: 1 cycle at 72°C - 2 min; 20 cycles 94°C - 20 sec, 56°C - 30 sec., 72°C 2 min; 1 cycle 72°C - 2 min; 1 cycle 60°C - 30 min (Techne, "FlexiGene" thermocycler, UK). A 5 µl aliquot has been removed from each sample and separated on 1.5% agarose gel to determine if the amplification reaction occurred. The remaining volume has been diluted from 10 to 50 times in relation to the visualised amplified sample intensity.

### Selective amplification

A mixture containing 1x buffer PCR, 0.2 ml dNTP, 2.0 ml MgCl₂, 0.625 µM *EcoRl* marked primer (+3), 0.625 µM *Msel* primer (+3) select, 0,2 U Hot master Taq polymerase (Eppendorf). 2mL 1:10 diluted sample of pre-selective PCR have been added to 8 µl final volume. PCR schedule was as below: 1 cycle 94°C - 2 min; 10 cycles 94°C - 20 sec, 66°C - 30 sec, 72°C 2 min. and for each cycle annealing temperature is lowered by 1°C; 25 cycles 94°C - 30 sec, 56°C - 30 sec, 72°C 3 min; 1 cycle 60°C - 30 min.

Initially 64 primer combinations have been tested. EcoRl primers have been marked using Cy5 fluorescent group. Adaptors sequences and used primer combinations for fAFLP analysis have been listed in Table 1. Amplification products have been stained on 1.2 % agarose gel containing ethidium-bromide in TBE 0,5x.

**Table 1**

| **Name** | **Sequenze** |
|---|---|
| *EcoRI* adaptor | 5'-CTCGTAGACTGCGTAAC-3' 3'-CTGACGCATGGTTAA-5' |
| *Msel* adaptor | 5'-GACGATGAGTCCTGAG-3' 3'-TACTCAGGACTCAT-5' |
| Primer used in preamplification | |
| *EcoRl* + 1-A | 5'-GACTGCGTACCAATTC + A-3' |
| *Msel* + 1-C | 5'-GATGAGTCCTGAGTAA + C-3' |
| Primer used in selective PCR | |
| *EcoRl* + 3-AGG | 5'-GACTGCGTACCAATTC + AGG-3' |
| *Msel* + 3-CTA | 5'-GATGAGTCCTGAGTAA + CTA-3' |

### Fluorescence Capillary Electrophoresis

Amplified fragments have been separated using Beckman Coulter, Inc. 8000 CEQ Genetic Analysis System genetic sequencer. 0,5 µL of selective amplification product have been mixed with 35 µl Sample Loading Solution (SLS) and 0,5 µl DNA Size Standards (600). CEQ separation conditions have been as below: denaturation at 90°C for 120 sec, injection for 30 sec at 1KV and separation at 5 KV for 55 min.

### Data analysis

Software for CEQ 8000 Beckman genetic analyser provided an electropherogram for every sample under study. The separation of detected peaks has been obtained with **1** nucleotide resolution in a range from 50 to 600 bp. In fAFLP analysis every peak can be present (1) or not (0) and data have been automatically analysed using a bio-information software (AFLP Dominant Scoring Software). Diversity level has been estimated as polymorphic percentage out of total obtained peaks.

### Results

### Physiological and enological characteristics of yeast according to the invention

### Saccharomyces bayanus DSM18477

- Generates spores on agar acetate
- Ferments and assimilates the following sugars: galactose, saccharose, maltose, raffinose and melibiose
- Does not grow on L-lysine as unique nitrogen source
- Generates H₂S at low-medium values (2 out of 1 to 5 scale)
- Does not grow in the presence of 3 g/l of cyclohexylimide
- Grows at 5°C in YPD medium
- Latency time: short
- Fermentation kinetics: fast and regular
- Sugar/ethanol yield: 14 g/l for 1° alcoholic grade
- Resistance to the alcohol: 16%
- Minimal inhibiting sodium metabisulfite concentration > 200 mg/l
- Production of volatile acids (g/l of acetic acid): 0,4-0,5
- Production of glycerol: 9-13 g/l

### Debaryomyces hansenii DSM18478

- Generates spores on agar acetate.
- Ferments and assimilates the following sugars: galactose, saccharose, maltose, raffinose
- Grows on L-lysine as unique nitrogen source
- Generates H₂S at low-medium values (2 out of 1 to 5 scale)
- Grows in the presence of 3 g/l of cyclohexylimide (Sigma)
- Grows at 5°C in YPD medium
- Minimal inhibiting sodium metabisulfite concentration 200 mg/I I
- Resistance to alcohol: 5%
- Produces β-glucosidase at very high values (5 out of 1 to 5 scale)

The current yeast although not being a good fermenting yeast, enhances variety and fermentation aroma. *Molecular characterisation and identification*

The molecular characterisation through the ribotyping allowed a preliminary identification of the species to be carried out, with subsequent confirmation by means of ALLEV 2.0 automated system.

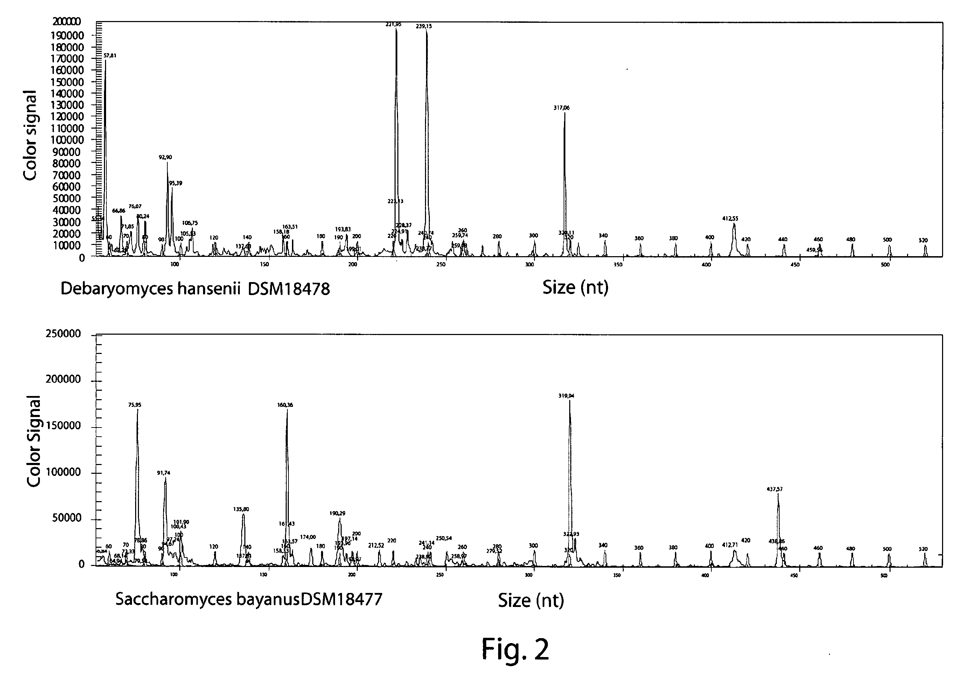

The molecular characterisation by means of AFLP fluorescence allowed strain identifying profiles, shown in Figure 2, to be obtained.

### EXAMPLE 2: Use of yeasts selected for the improvement of spumante produced according to classic method and analysis of the effects on olfactively active aromatic component

### MATERIALS AND METHODS

### Sampling

The samples (Test, T1, T2, T3) (Witness: commercially available *Saccharomyces bayanus* yeast EC1118, Lallemand, Canada; T1: DSM18477; T2: DSM18477+DSM18478; T3:

EC1118+DSM18478), maintained at temperature of 4°C, have been opened and immediately analysed (titerable acids, volatile acids, alcoholic degree, gas-chromatographic analysis, olfactometric analysis, sensory analysis) at laboratories of Tecnologie Agroalimentari DOFATA, Facoltà di Agraria Università di Catania.

### Extraction of volatile component

The extraction of volatile component has been carried out by means of SPME (Solid Phase Micro Extraction) technique on 20 ml aliquot of sample. After the sample conditioning step in graduated vial under agitation for 30 min at 35°C, a 50/30 µm DVB/CAR/PDMS (Supelco) fiber, previously conditioned (270°C for 1 hour), has been inserted within the sample head space and equilibrated for 15 min, then desorbed for 5 min in gas-chromatograph injector (250°C) for quantitative (GC/FID) and olfactometric (GC/O) analyses or subjected to GC/MS for qualitative identification.

Before the sample analysis, the reliability of the selected fibre for SPME analysis has been tested, i.e. the maximum number of perfectly repeatable successive extractions on the same matrix. The fiber, after the conditioning (270°C for 1 hour) and a blank test, has been used for n successive Witness sample extractions taking as evaluation parameters areas of 8 selected aromatic components; after the elimination of data relating to first two analyses, considered as conditioning the fibre to the champion, it has been observed that all variability coefficients (CV%) of 8 components were maintained between 4 and 16%, more than acceptable values, until the seventeenth analysis as reported in Table 2. Therefore It has been chosen to use every fibre, after the conditioning, 1 blank test and 2 null tests, for a total of 15 analyses.

Then the analyses of the 4 spumante samples have been carried out.

**Table 2**

| **Component** | **CV %** | | |
|---|---|---|---|
| Isobutanol | 7.52 | | *Lines for calibration lines and quantitative calculation (method of external standards)* |
| Iso-amyl alcohols | 6.61 | | |
| ethyl hexanoate | 4.60 | | |
| ethyl lactate/1-hexanol | | 8.85 | |
| Ethyl octanoate/acetic acid/iso-amyl hexanoate | | 10.08 | |
| ethyl decanoate | | 11.44 | |
| diethyl succinate | | 15.78 | |
| 2 phenyl ethanol | | 16.68 | |

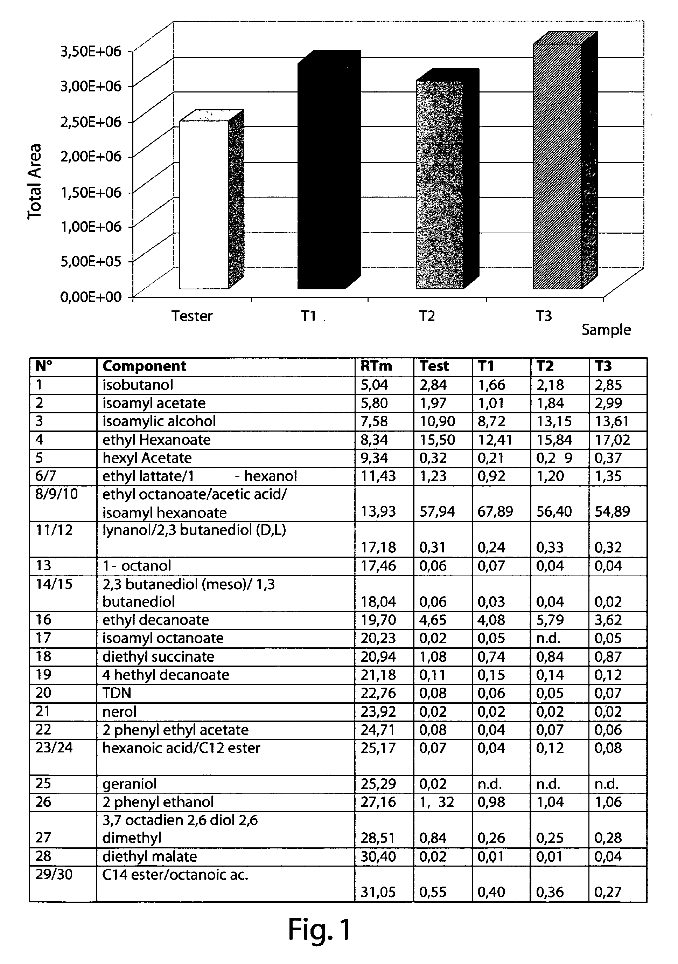

A solution model according to the obtained average values from base analyses on the samples (5 g/l of tartaric acid, 7 g/l of saccarose, 13.5 alcohol %) has been prepared. From the model solution a set of 5 vials (20 ml) at variable concentrations for everyone of the standard components in our possession has been prepared and analysed in the same extraction conditions used for the samples (previously described). For every component as lower and upper limits have been considered respectively the concentrations corresponding to the minimal and maximum areas found for analysed samples (Figure 1). For every standard component it has been therefore constructed a calibration line to be used subsequently for the calculation of the concentrations in the samples (minimal and maximum concentrations (ppm) of standard in model solution; Table 3).

**Table 3**

| **Component** | **Min - Max** | **R²** |
|---|---|---|
| Iso-amyl acetate | 0.05 - 2 | 0.9960 |
| Ethyl hexanoate | 0.25 - 2 | 0.9820 |
| Hexyl acetate | 0.005 - 0.06 | 0.9960 |
| Ethyl lactate/1-hexanol | 0.5 - 2 | 0.9874 |
| Ethyl octanoate | 0.25-0.6 | 0.9912 |
| Linalol | 0.005-0.06 | 0.9678 |
| 1-octanol | 0.005-0.06 | 0.9853 |
| Ethyl decanoate | 0.005-0.06 | 0.9752 |
| Diethyl succinate | 0.5-2 | 0.9918 |
| Nerol | 0.005-0.06 | 0.9839 |
| 2 phenyl ethyl acetate | 0.005-0.06 | 0.9796 |
| Geraniol | 0.005-0.06 | 0.9871 |
| 2 phenyl ethanol | 5-10 | 0.9963 |

As it can be observed, r² relating to lines of each listed component is > 0.9678, as an indication of response linearity of the fibre to the compound with the concentration variation. It is moreover important to emphasise that this linearity has been demonstrated from a minimal of 0.005 ppm (for example for linalol, ethyl decanoate etc) to a maximum concentration of 10 ppm (2-phenyl ethanol); for concentrations higher than 10 ppm, as for isobutyl alcohol or iso-amyl alcohols, a linear trend of the fibre response has been observed until 15 ppm and then flattening from 20 to 50 ppm, probably due to a saturation limit of the fiber.

### GC/FID - GC/O analyses

GC SHIMADZU GR-17 AAF equipped with CP-WAX 52 CB (50 m, 0,25 ml i.d., 0,25 µm film thickness) column operating according to following conditions has been used: carrier helium at constant speed (35 cm/sec); split 1:10 ratio; initial isotherm at 70°C for 3 min; temperature variation 4°C/min; final isotherm at 220°C for 20 min. Column exit flow has been equally subdivided (1:1) using Y means (VSOS - SGE - Alltech Italy s.r.l) between FID (280°C) and the sniffing port equipped with air humidifier (30 ml/min).

Olfactometric analyses, performed according to OSME method, have been carried out by a panel (2 men and 2 women, average age 25 years) consisting of 4 judges, previously employed and opportunely trained as described in Guarrera et al., 2005.

Before the sample analysis, three preliminary analysis tests, in order to verify the judge ability to detect and recognise odorous standards (p<0,1) have been carried out.

Descriptors and intensity values (non continuous scale from 1 to 7) orally attributed to every perceived odorous molecule were recorded by another operator, while the persistence of olfactive *stimuli* was recorded simultaneously by means of the pressure duration of a gas-chromatograph interfaced push-button. Every judge replicated the analysis of every sample (30 min time length) 3 times in the same day with intervals of at least 1 hour among different sessions.

### GC/MS analysis

GC/MS SHIMADZU QP 5050 instrument operating at the following conditions has been used: source El - 70 eV; column CP-WAX 52 CB with same characteristics as for GC/FID - GC/O analyses; carrier helium (2 ml/min); injector temperature 220°C; initial temperature 70°C, variation 4°C/min; final isotherm 220°C for 15 min; temperature 200°C; split ratio 9. The identification of aromatic, odorous or not, molecules, has been carried out based on average retention times, fragmentation patterns, and odorous characteristic of our standards.

### Sensory analysis

All sensory analyses have been carried out in UNI ISO8589 certified DOFATA laboratories of the Facoltà di Agraria of the Università di Catania (Italy).

The first step of the sensory analysis schedule has involved a set of triangular tests (UNI U590A2520, 2001) aimed to estimate the detectability of the 3 experimental samples in relation to a commercially available product by a panel of 30 trained and usual wine consumer judges. Samples (30 ml) have been proposed in glasses with plastic disc shaped coverings at temperature of 15°C. All the triangular tests have been carry out during the same day (from 10:00 to 12: 00) in individual white light illuminated box. Then quantitative descriptive analysis (ISO/FDIS 13299, 2002), aimed to define the sensory profile of the 4 samples, has been carried out through a list of 16 common descriptors resulting from an appropriate preliminary sample session (free vocabulary method; 70% minimal citation). Selected descriptors have been the following: 2 relating to the colour (yellow amber), 8 relating to the aroma (fruity, citruses, mature apple, dried fruit, sultanine grape, floral, biting spicy , sweet spicy), 3 relating to the taste (sour, sweet, bitter), 2 relating to the body (full, balanced), 1 relating to the kinesthetic feeling (biting). 10, all usual wine consumer, judges (3 men, 7 women, average age 27 years), have been selected based on their ability to memorise and recognise 90% of the 54 aromas contained in the training kit "Le *Nez du Vinr"* (by Jean Lenoir, Editions Jean Lenoir). Every judge has been asked to quantify the intensity of aforesaid descriptors by means of nine point discontinuous scale; every judge has repeated the evaluation on the same sample three times during the same day with at least of 2 hour test interval.

Statistical elaboration of the obtained sensory data has been carried out by means of Three-way ANOVA and LSD multiple range test (p<0,05) using Statgraphics 5,1 Plus version software for Windows (Manugistics Inc., Rockville, MD, USA).

### RESULTS

The first comparison among the 4 samples has been carried out based on the total aromatic content expressed as sum of the areas of the 30 identified aroma components (Figure 1). It is apparent that the Witness sample is characterised by clearly lower total aromatic content than the others 3 samples and, among these, sample T3 is higher than T1 and at last T2. In the light of this first difference the calculation of percentage distribution for individual aromatic components in each of the 4 samples, in order to estimate which are the compounds mainly contributing to the total differences just described (Figure 1, table). The table reported in Figure 1 shows the percentage distribution of the aromatic components detected in the 4 samples according their average retention times. 30 listed components have been identified in all the four samples, demonstrating the unique used cultivar and same enological adopted procedures. Some components, as for example ethyl lactate and 1-hexanol or linalol and 2,3 butane diol (D, L), are separately numbered but considered as unique peak, because not separable in used gas-chromatographic conditions. The first apparent consideration is the following: 4 compounds are sufficient, i.e. ethyl octanoate (most representative absolutely), ethyl hexanoate, iso-amyl alcohols and ethyl decanoate, to reach approximately 90% of the total aromatic percentage distribution in all the four samples, not showing moreover substantial differences among them. Taking in consideration all the others components, approximately remaining 10%, only meaningful differences regard the percentage distribution of iso-amyl acetate approximately at 3/1 ratio between T3 and T1, 3,7 octadien-2,6-diol-2,6-dimethyl in percentage approximately 3 times higher in the Witness than other 3 samples and the peak of hexanoic acid/ C12 ester at 3/1 ratio between T2 and T1.

Other small differences, detectable for example for diethyl malate or iso-amyl octanoate, are not to be considered meaningful because at excessively small percentages.

The percentage distribution can be considered only representative of the effective content of an aromatic component, but it really does not quantify the same. Therefore in order to calculate the effective concentration (ppm) it has been necessary to use the calibration lines constructed for the components detected in SPME aromatic extract of the samples (see materials and methods).

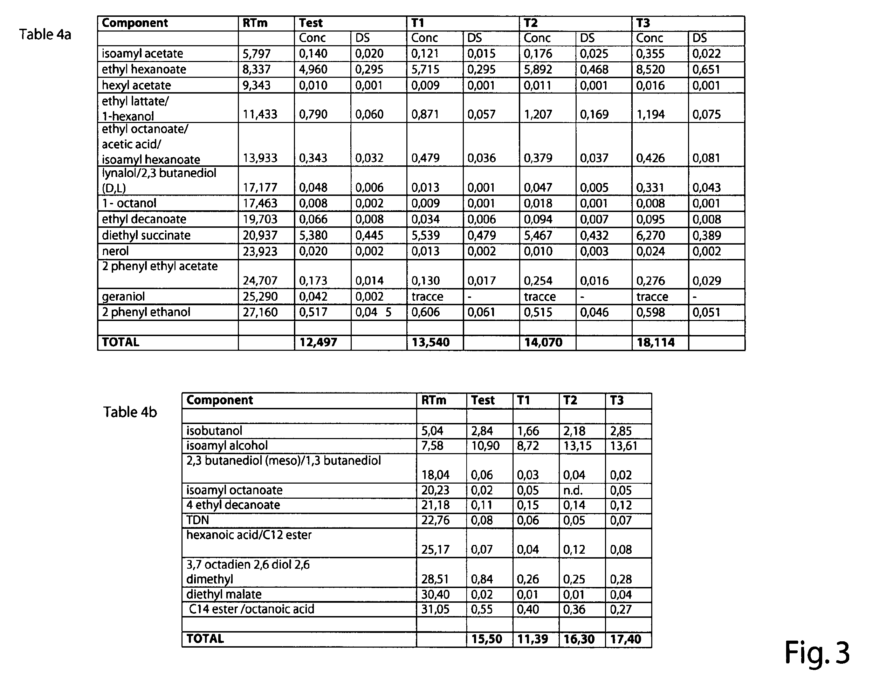

At this point, all the aromatic components identified and grouped in the table of figure 1, have been subdivided in two subgroups, in the first of which (Figure 3, Table 4a) all the quantifiable components have been inserted (concentration in ppm) according to the external standard method, while in the second (Figure 3, Table 4b) have been inserted the remaining quantifiable components using percentage distribution of the areas have been inserted. It is important to emphasise that these last ones constitute at maximum 17% (sample T3) of the total aromatic area and are principally represented by individual iso-amyl alcohols (8-13%).

As to the concentrations of the components reported in Table 4A (Figure 3), it is possible to do the following considerations: the effective total aromatic content of the four analysed samples increases progressively from the witness sample to T2 (12.497 - 13.540 - 14.070), and this increment appears still more apparent in sample T3 having a value of 18.114 ppm; geraniol is quantifiable exclusively in the witness sample unlike the others where it is present only in traces; the peak corresponding to elution of linalol and 2,3 butane diol (D, L) is remarkably reduced in T1 sample similarly to ethyl decanoate, while 1-octanol is noteworthy due to doubled concentration in the T2 sample in comparison to others. Except for these small differences, T3 sample appears surely the most distinguishable from the quantitative point of view showing various components at clearly higher concentrations than average of the others 3 analysed samples, and particularly iso-amyl acetate (0.355 ppm), ethyl hexanoate (8.520 ppm), linalol/2,3-butane diol (D, L) (0.331 ppm), diethyl succinate (6.270 ppm) and 2-phenyl ethy acetate (0.276 ppm).

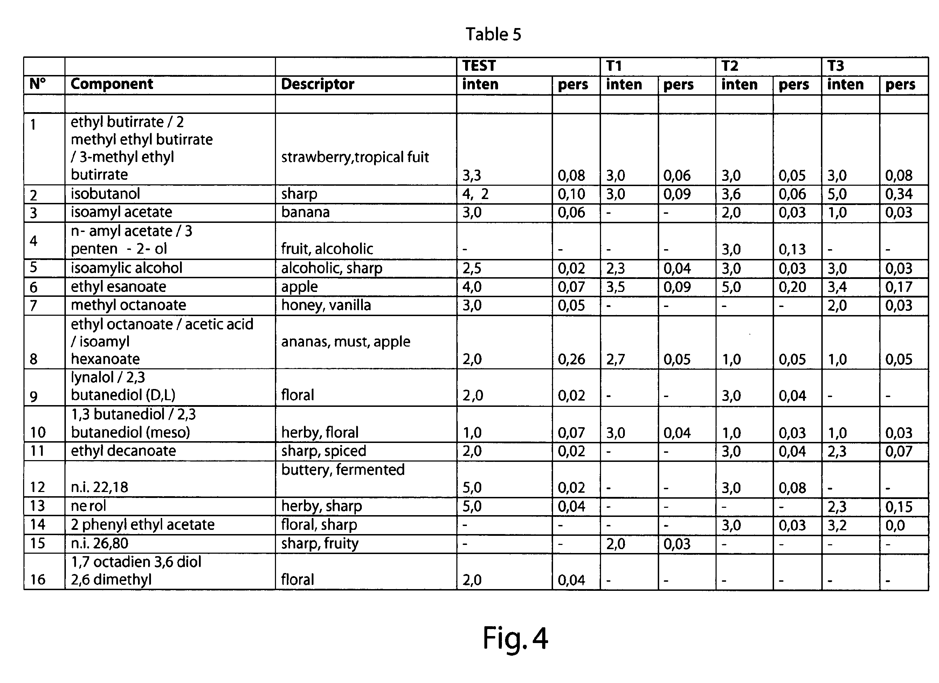

As to the olfactometric analyses, in Table 5 (Figure 4) aromatic compounds identified as olfactively active are reported, the descriptor/s associated thereto, and the corresponding intensity and persistence values.

Obviously not all the aromatic and reported in the table of Figure 1 compounds proved to be odorous in reason of their concentration in the samples and relative perception threshold. Viceversa, some compounds like ethyl butyrate, methyl octanoate, or n.i. components (22.18 RTm and RTm 26.80), although not quantifiable in used chromatographic conditions and therefore not reported in the table of Figure 1, however show a good olfactive impact.

The first apparent aspect is that, unlike quail-quantitative gas-chromatographic analysis, olfactometric analyses point out 4 products with enough heterogeneous aromatic/olfactive profiles, in fact, out of 16 total odorous compounds characterised, only 6 (ethyl butyrate, iso-butanol, iso-amyl alcohols, ethyl hexasanoate, ethyl octanoate and 1,3 butane diol) are common in all the four samples. This is easily explicable considering high contents of said compounds (Table 4a - 4b, Figure 3). There are excluded ethyl butyrate, which is eluted under ethanol peak together with other chemically similar compounds, and 1,3 butane diol co-eluting with 2,3-butane diol (meso); both compounds are easily detectable since characterised by a very low perception threshold.

Viceversa, only 3 olfactive notes characterises a single sample in comparison to all the others: i.e. floral note resulting from 1,7-octadien-3,6-diol-2,6-dimethyl in the Witness (13 total odorous notes), biting/fruity note from n.i. RTm 26,80 component in T2 (7 total odorous notes), and fruity/alcoholic note from co-presence of n-amyl acetate/3-penten-2-ol in T2 sample (12 total odorous notes). Only in T3 sample, have not been detected characteristic odorous notes of the product among 11 totally detected by the judges.

It is important to point out the correspondence existing between the greater part of the olfactometric responses and quantitative results (Table 4A, Figure 3), in fact, with the exception of the common and abundant odorous components in all the samples as above evidenced, for example nerol is perceived only when it is present in higher concentrations (Witness and T3); the same could be true for 2-phenyl ethyl acetate or ethyl decanoate, not detected when present at concentrations lower than medium perception threshold.

A final consideration concerns linalol, odorous component detected with its characteristic floral note only in witness and the T2 samples. Its absence in the aromatic/olfactive profile of the T1 surely results from its low concentration (0.013 ppm) in the same, while the fact that it is not detected in T3 sample, although the peak corresponds to clearly higher concentrations (0.331 ppm), is probably the consequence of an increment of the odourless compound co-eluting with linalol, i.e. 2,3-butane diol (D, L).

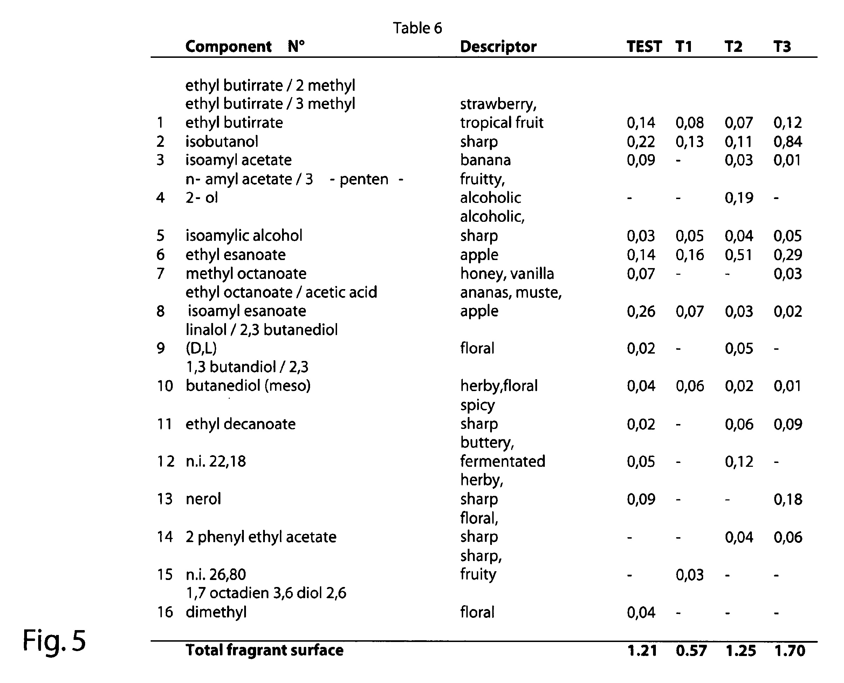

Finally from intensity and persistence data of individual odorous compounds have been deducted AUC values (area under curves), to be used as parameter in order to quantify and compare the total aromatic-olfactive impact of the 4 spumante samples (Table 6, Figure 5). As a consequence T1 sample shows the lowest odorous surface (0.57), Witness and T2 samples show similar values (1.21-1.25 respectively), while T3 is the best product as to aromatic-olfactive impact (1.70).

Above considerations are reported in Figures 2a - 2b - 2c - 2d, wherein the graphical representation offers a more immediate visualization of the existing correspondences between the olfactometric responses, represented in the 4 osmograms, and analytical data, extrapolated from corresponding experimental chromatograms.

Passing to consider the sensory evaluations, the results of the triangular tests have shown that all and the three experimental samples (T1, T2, T3) have reached detection limit such that the same can be considered statistically different (p ≤0.05) in comparison to the witness. These differences have been estimated in the successive descriptive analysis tests and thus obtained data have been statistically elaborated (see. materials and methods section).

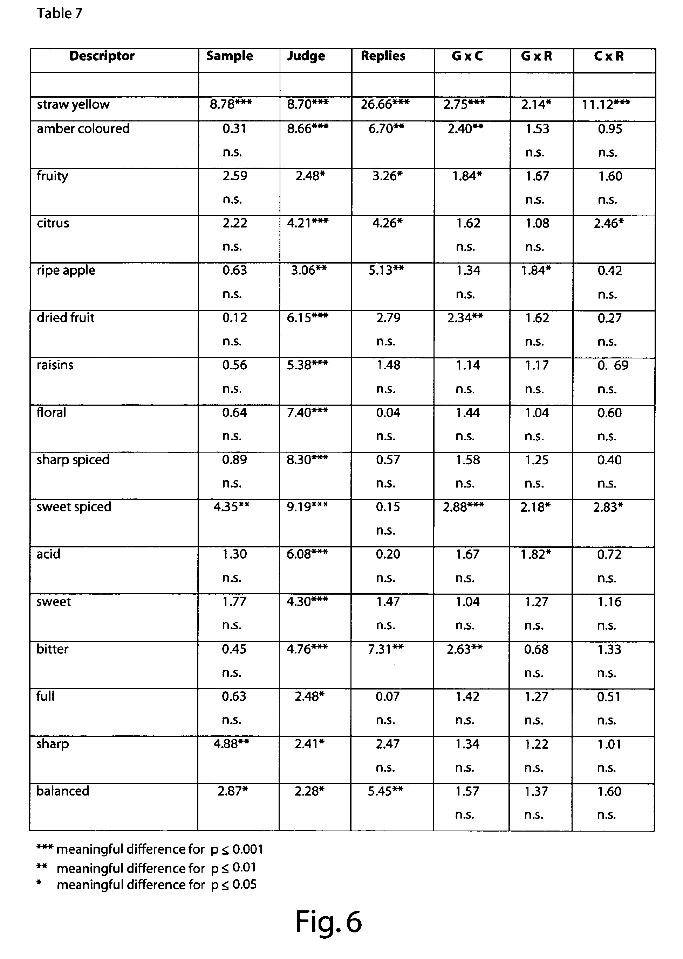

Three-way ANOVA analysis allowed to estimate not only for which variable (descriptors) factors (samples) result meaningfully different from each other, but also to be able to estimate Judges x Samples (G x C = index of the judge behaviour on average values attributed to the samples), Judges x Replicates (G x R = index of the judge repeatability during the replicates), Samples x Replicates (C x R = index of characteristic homogeneity of each sample during replicates) interactions. Analysis results are reported in Figure 6 in Table 7 and can be reassumed as below: samples proved to be significantly different for the descriptors of yellow amber colour (p ≤ 0.001), aroma sweet spicy (p ≤ 0.01), biting (p ≤ 0.01) and balanced (p ≤ 0.05); judges have shown significant differences among them for all and 16 descriptors (p ≤ 0.001 - p ≤ 0.05); replicates have shown a good repeatability with the exception of some descriptors (for example. yellow amber, bitter, mature apple etc); interactions G x C, G x R and C x R have evidenced respectively a good discrimination ability of the samples by the judges, the reliability of the panel and a good sample homogeneity during the replicates.

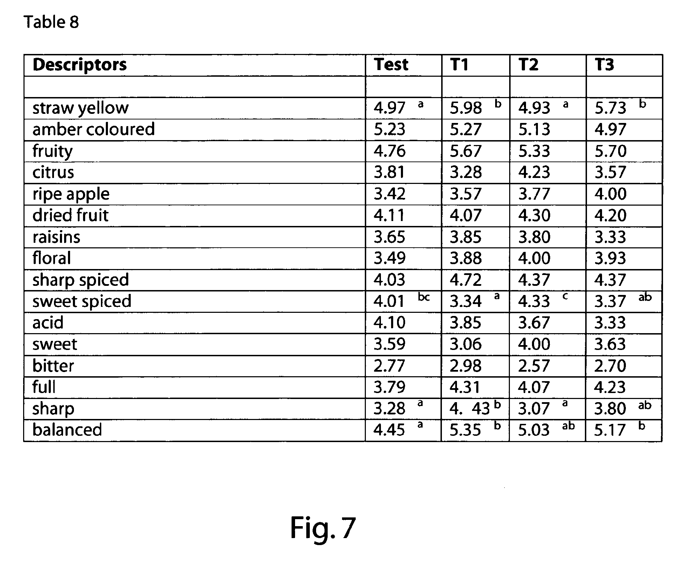

At last the multiple range test (Figure 7, Table 8) has allowed to estimate, based on LSD calculation (Least Significance Difference), the distribution of the differences existing among the 4 samples. Therefore the yellow amber colour appears to be a descriptor symbol of witness and T2 in comparison to T1 and T3 samples; analogous consideration can be made for biting and balanced descriptors, while the unique descriptor able to differentiate among all the 4 samples is that corresponding to the pleasant aroma of sweet spices.

### BIBLIOGRAPHY

- Steinhart H., Stephan A., Bücking M. (2000). Journal of High Resolution Chromatography, 23 (7/8), 489-496.
- Dravnieks A., O'Donnel A. (1971). Journal of Agricultural and Food Chemistry, 19, 1049-1056.
- Guadagni D.G., Okano S., Buttery R.G., Burr H.K. (1996). Food Technology, 20, 518-521.
- Rose M.F., Winston F., Hieter P. (1990). A Laboratory Course Manual. Cold Spring Harbor Laboratory Press, Cold Spring Harbor, New York.
- Mortimer RK, Romano P, Suzzi G, Polsinelli M. (1994). Yeast. 10, 1543-52.
- Van der Walt J.P., Yarrow D. (1984). The yeasts a taxonomic study, edz. Kreger van Rij, Elsevier Science Publishers B.V., Amsterdam.
- McCullough M.J., Clemons K.V., McCusker J.H., Stevens D.A. (1998). J. Clinic. Microbiol. 4, 1035-1038.
- Dresler-Nurmi, A., Terefework, Z., Kaijalainen, S., Lindström, K. and Hatakka, A. (2000). J. Microbiol. Methods 41, 161-172.
- Barros Lopes M., Rainieri S., Henschke P. A.e Langridge P. (1999). International Journal of Systematic Bacteriology 49, 915-924.
- Borst A., Theelen B., Reinders E., Boekhout T., Fluit A.C., e Savelkoul P.H.M. (2003). JCM .41.4.1357-1362.
- Guthrie C.Y. & Fink G. R. (1991) Academic Press. Inc., NY.
- Guarrera N., Campisi S., Nicolosi Asmundo C. American Journal of Enology and Viticulture. 56:4; 394-399 (2005).

## Claims

1. *Saccharomyces bayanus* strain, deposit No. DSM18477.

2. *Debaryomyces hansenii strain,* deposit No DSM18478.

3. Combination of *Saccharomyces bayanus* DSM18477 and *Debaryomyces hansenii* DSM18478.

4. Combination of *Saccharomyces bayanus* DSM18477 or *Debaryomyces hansenii* DSM18478 with another commercially available yeast.

5. Use of at least one of the two strains as defined in claim 1 or 2 or their combination as defined in claim 3 or 4 in a yeast re-fermentation and/or autolysis process to obtain a re-fermented drink.

6. Use according to claim 5, wherein said process it is the champagnisation and said drink is spumante.

7. Use of at least a yeast strain as defined in claim 1 or a yeast combination as defined in claim 3 or 4 in a yeast fermentation process to obtain a fermented drink selected from the group consisting of cider, fruit juice, beer, wine, raisin wine and sake.

8. Mixture comprising the following components:
- at least one of the two strains as defined in claim 1 or 2 or their combination as defined in claim 3 or 4;
- sugar, preferably cane sugar;
- base wine.

9. Mixture according to claim 8, wherein said sugar is at a concentration of 20-25 g/l, preferably 24 g/l.

10. Fermentation process to obtain a re-fermented drink, **characterised in that** it comprises a bottle re-fermentation and autolysis step using a mixture, as defined according to each of claims 8-9, at a temperature from 8 to 16°C for at least 8-10 months.

11. Fermentation process according to 10, wherein said temperature is 10°C.

12. Process according to each of claims 10-11, wherein said fermentation process is champagnisation process and said re-fermented drink is spumante.

13. Re-fermented drink obtainable according to champagnisation process according to each of claims 10-11.

14. Spumante obtainable by champagnisation process as defined in claim 12.

15. Fermentation process to obtain a fermented drink, **characterised in that** it comprises a fermentation step of a fermentable base by using at least a yeast strain as defined in claim 1 or a yeast combination as defined in claim 3 or 4, to obtain a fermented drink selected from cider, fruit juice, beer, wine, raisin wine and sake.

16. Process according to claim 15, wherein said fermentable base it is chosen from the group consisting of malt, must, fruit juice, rice.

17. Fermented drink obtainable using the champagnisation process according to each of claims 15-16.
